# EUROPEAN PATENT SPECIFICATION

(11) **EP 0 728 728 B1**
(45) Date of publication and mention of the grant of the patent: **05.04.2000**
(21) Application number: 96200439.6
(22) Date of filing: 20.02.1996
(51) Int. Cl.: C07C 51/41, C07C 53/122, C07C 59/105, B01J 19/18

(54) **A method for the production of calcium salts of weak acids**
Verfahren zur Herstellung von Calciumsalzen der schwachen Säuren
Procédé de production des sels de calcium d'acides faibles

(30) Priority: 23.02.1995 IT MI950342
(43) Date of publication of application: 28.08.1996
(73) Proprietor: VOMM CHEMIPHARMA S.r.l., 20121 Milano (IT)
(72) Inventor: Vezzani, Corrado, 20146 Milano (IT)
(74) Representative: Vannini, Torquato

(56) References cited:
- EP-A- 0 304 831
- AT-A- 368 910
- GB-A- 1 084 724

## Description

The present invention relates generally to the commercial production of calcium salts of weak acids, that is to say acids having a pH > 3.5, including organic and inorganic acids.

More particularly, but not exclusively, the invention is concerned with a method for the production of calcium salts of mineral acids such as phosphoric acid and carboxylic acids such as propionic and gluconic acids which, it is well known, are widely used in many technical fields.

Thus, for example, calcium phosphates are widely used in the production of fertilisers, in raising agents for human food products, in animal feedstuffs and in the pharmaceutical field.

Calcium propionate is used particularly, but not exclusively, in animal feedstuffs.

Calcium gluconate is used mainly in the pharmaceutical industry as a calcium additive.

It is known that, at least on the industrial scale, the calcium necessary for the salification of weak acids, particularly the acids mentioned above, is provided by the use of hydrated lime (slaked lime) and/or calcium oxide (quick lime), which products are obtained by the thermal decomposition of calcium carbonate.

More particularly, calcium carbonate is calcined or baked at high temperatures, of the order of 850 to 900°C, forming calcium oxide. Slaked lime is then made from this by hydration, with the evolution of a great deal of heat which is totally wasted.

Hence, in view of the energy expenditure needed to obtain them, calcium oxide and slaked lime may be thought of as "noble" products relative to the starting calcium carbonate.

It is this "nobility", this considerable energy expenditure, which is a first recognised disadvantage of the prior art.

A further disadvantage is the time required for the reactions between the weak acids and the slaked lime; it is in fact known that the reaction time varies from several hours to many hours according to the nature and conditions in which the starting materials are provided and used.

Moreover, still with reference to the prior art, in order to facilitate the salification of the weak acids, that is, to obtain the best possible contact between the reagents, in many cases it is necessary to dilute the slaked lime with a great deal of water which it is subsequently necessary to remove, requiring further considerable expenditure of energy.

These problems could be overcome completely if the source of calcium used for the salification of the weak acids under consideration were calcium carbonate or, in other words, if one were able to react calcium carbonate directly with the selected weak acid.

Many attempts have been made to do this but none of them has resulted in an industrially significant and acceptable result, most probably due to the reactivity of calcium carbonate which is known to be poor (or almost nil).

European patent application No. 0 304 831 discloses a continuous process for preparing salts of fatty carboxylic acids, which involves reacting fatty carboxylic acids with, i.a., solid carbonates of metals such as e.g. calcium. In this process the acids are mixed in a molten state with the solid metal carbonate in a tubular reactor containing a rotor provided with a mixing spiral.

The problem at the root of the present invention is therefore to provide a method for the industrial production of calcium salts of weak acids, of the type specified, which uses calcium carbonate directly so as to overcome all the disadvantages mentioned above with reference to the prior art.

This problem is solved according to the invention by a method which is characterised in that it includes the successive steps of:
- continuously supplying substantially anhydrous powdered calcium carbonate and a substantially atomized weak acid to a continuous salification reactor having a tubular cylindrical body with an inner wall heated to a temperature of between 100 and 450°C and which a bladed rotor is rotatably supported,
- centrifuging the calcium carbonate and the weak acid to form an extremely turbulent, annular fluidized bed,
- advancing the annular fluidized bed through the continuous salification reactor and simultaneously and continuously administering to it the heat necessary to cause the salification of the weak acid through the hot wall,
- continuously discharging from the reactor the products of the salification reaction, including carbon dioxide, water vapour and the calcium salt of the weak acid.

In the finely dispersed conditions with high turbulence in which the calcium carbonate and the selected weak acid are maintained in the fluidized bed, which is formed mechanically in the continuous salification reactor, intimate, diffuse and total contact is ensured.

It has been found, surprisingly, that, in these conditions, with the continuous administration of an appropriate, quantity of heat and the simultaneous removal of the gaseous reaction products, not only can the reaction be triggered readily but it also goes to completion in a very short period of time.

Thus, for example, it has been found that most of the weak acid is salified with stay times in the continuous reactor of the order of one to two minutes.

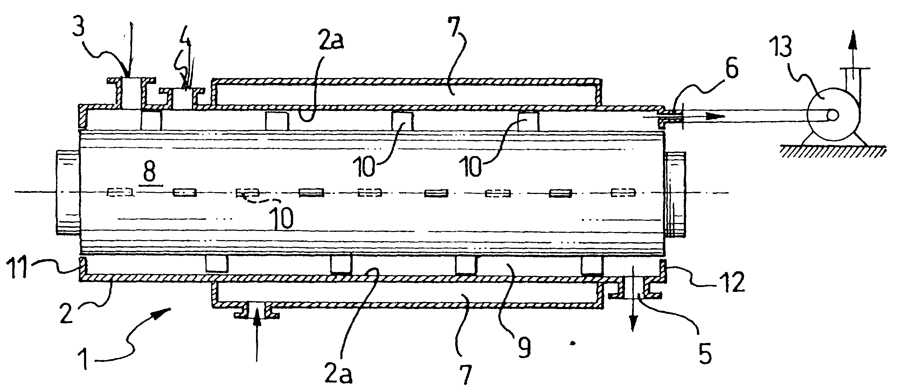

Further advantages and characteristics of the invention will become clearer from the description which follows of several embodiments of the method for the production of calcium salts of weak acids, made with reference to the appended drawing, given purely by way of non-limitative example, which shows schematically a continuous salification reactor according to the invention.

With reference to the drawing, a continuous salification reactor is generally indicated 1 and comprises a cylindrical tubular body 2 arranged with its axis horizontal, closed at opposite ends by end walls 11, 12 in which there are inlet apertures 3, 4 for the input of the reagents at one end and outlet apertures 5, 6 for the salt produced and for the removal of the gaseous reaction products (water vapour and carbon dioxide) at the other end.

The tubular body 2 has a jacket 7 traversed by a conventional heating fluid which is able to maintain the inner wall 2a of the tubular body 2 at a temperature of between 100 and 450°C.

A bladed rotor 8 is rotatably supported in the tubular body 2 of the continuous salification reactor of the invention and has associated drive means (not shown since they are conventional) for rotating it at a speed which can vary from 300 to 1500 revolutions per minute. The rotor 8 defines, with the heated inner wall 2a mentioned above, a tubular interspace 9 which constitutes the reaction chamber proper of the continuous reactor 1 and is in fluid communication through the aperture 6 with a conventional suction unit 13 outside the continuous reactor 1. The blades 10 of the rotor 8 project radially from the rotor itself and terminate close to the hot inner wall 2a of the continuous reactor and are arranged in a helix around the rotor itself.

In order to produce calcium salts of weak acids, independent flows of substantially anhydrous powdered calcium carbonate and atomized weak acid are supplied continuously to the continuous salification reactor 1 through the apertures 3 and 4 respectively.

In the continuous reactor, the inner wall 2a is kept at a temperature (from 100 to 450°C) which is predetermined according to the nature of the reactants, their initial conditions of purity, etc. Moreover the rotor 8 is rotated at a speed sufficient to centrifuge the materials supplied to the continuous reactor towards and against the hot wall 2a thereof, forming an annular fluidized bed.

The orientation of the blades 10 of the rotor 8 is such that the materials are thrust (always in the form of an annular fluidized bed) towards the outlet apertures.

Immediately at their inlet to the continuous reactor, the calcium carbonate and the weak acid selected are centrifuged so as to form the said annular fluidized bed in which they are finely dispersed, in extremely turbulent conditions.

In these conditions and due to the heat supplied through the hot wall 2a with which the fluidized bed is in constant contact, the salification reaction is triggered immediately and goes to completion with the simultaneous liberation of carbon dioxide and water in vapour form. The water and carbon dioxide are removed (withdrawn) from the continuous reactor immediately they form.

After a very short stay time, of the order of one to two minutes, the calcium salt of the selected weak acid is discharged from the aperture 5.

### EXAMPLE 1

### Production of Calcium Phosphates

Substantially anhydrous, powdered calcium carbonate was supplied continuously to the continuous salification reactor 1 at a flow rate of 100 kg/hour together with an atomized flow of aqueous phosphoric acid.

The bladed rotor 8 of the continuous reactor 1 was rotated at a speed of 1000 revolutions per minute while the hot wall 2a was kept at a temperature of from 220 to 300°C, preferably between 260° and 280°C. Almost at the same time as the reagents were introduced, a gaseous flow was withdrawn from the reactor which consisted essentially of carbon dioxide and water vapour, thus showing that the salification reaction had started immediately the reagents were fed into the continuous reactor.

After a stay time of about one minute in the continuous reactor, substantially anhydrous, powdered calcium phosphate started to be discharged from it. By varying the temperature of the wall 2a and the concentration of the phosphoric acid in the aqueous solution supplied to the continuous reactor from 58% to 76% and by varying the CaCO₃/H₃PO₄ ratio, the product discharged varied from:
Dicalcium phosphate dihydrate with a P₂O₅ equivalent of 17%
Monocalcium phosphate monohydrate with a P₂O₅ equivalent of 22%
Mono-dicalcium phosphate with a P₂O₅ equivalent of 19%

### EXAMPLE 2

### Production of Calcium Gluconate

Substantially anhydrous powdered calcium carbonate was supplied to the continuous salification reactor 1 of the invention at a rate of 100 kg/hour together with an atomized 60% aqueous gluconolactone solution.

The bladed rotor 8 of the continuous salification reactor was rotated at a speed of 1200 revolutions per minute while the temperature of the inner hot wall was kept at a constant value of 250°C.

A gaseous flow constituted essentially by carbon dioxide and water vapour was withdrawn from the reactor almost simultaneously with the introduction of the reagents.

After a stay time of two minutes, a continuous flow of calcium gluconate was discharged from the continuous salification reactor.

It should be noted that, when gluconolactone is used, the lactone is converted initially to gluconic acid and the salification reaction occurs in a subsequent step, although still continuously.

The product (calcium gluconate) discharged from the continuous reactor had a residual moisture content which can be removed when desired by conventional drying.

### EXAMPLE 3

### Production of Calcium Propionate

Atomized propionic acid was supplied continuously at a flow rate of 100 kg/hour with powdered calcium carbonate in the stoichiometric ratio to the continuous salification reactor 1, the inner wall 2a of which was kept at a temperature of 130 to 150°C, with the bladed rotor 8 rotated continuously at a speed of 980 revolutions per minute.

A gaseous flow constituted essentially by carbon dioxide and water vapour was withdrawn from the reactor almost simultaneously with the introduction of the reagents.

After a stay time of about one minute, calcium propionate started to be discharged continuously from the reactor.

Again in this case the product leaving the continuous reactor was subjected to a further drying step to remove its residual moisture content completely.

The advantages achieved by the invention may be summarised as follows:
- a very economic method of production (both due to the direct use of calcium carbonate and the saving in energy),
- very high productivity with a high yield,
- constancy of the physico-chemical characteristics of the product obtained,
- absence of dust,
- possibility of varying the grain size of the product at will,
- very homogenous and regular product content.

## Claims

1. A method for the continuous production of calcium salts of weak acids, characterised in that it comprises the successive steps of:
- continuously supplying substantially anhydrous, powdered calcium carbonate and a substantially atomized weak acid to a continuous salification reactor having a tubular cylindrical body with an inner wall heated to a temperature of between 100°C and 450°C and which a bladed rotor is rotatably supported,
- centrifuging the calcium carbonate and the weak acid to form an extremely turbulent, annular fluidized bed,
- advancing the annular fluidized bed through the continuous salification reactor and simultaneously and continuously administering to it the heat necessary to cause the salification of the weak acid to occur through the hot wall,
- continuously discharging from the reactor the products of the salification reaction, including carbon dioxide, water vapour and the calcium salt of the weak acid.

2. A method according to Claim 1, characterised in that the weak acid is selected from the group comprising phosphoric, propionic and gluconic acids.

3. A method according to Claim 2, characterised in that the weak acid is phosphoric acid in 58-76% aqueous solution, the inner wall of the continuous salification reactor being heated to a temperature of between 220°C and 300°C.

4. A method according to Claim 2, characterised in that the weak acid is propionic acid and the temperature of the inner wall of the continuous salification reactor is between 130°C and 150°C.

5. A method according to Claim 2, characterised in that the weak acid is gluconic acid and the inner wall of the continuous salification reactor is kept at a temperature of 250°C.

6. A method according to Claim 5, characterised in that the gluconic acid is supplied in the form of a 60% aqueous gluconolactone solution.

7. A continuous reactor for the production of calcium salts of weak acids starting from powdered calcium carbonate, comprising:
- a cylindrical tubular body (2) closed at opposite ends (11, 12) at which there are inlet apertures (3, 4) for the introduction of the reagents and discharge apertures (5, 6) for the reaction products,
- at least one jacket (7) traversed by heating fluid for controlling the temperature of the inner wall (2a) of the continuous reactor (1) to values of between 100°C and 450°C,
- a bladed rotor (8) rotatably supported in the tubular body (2) and defining, with the heated inner wall (2a), a reaction chamber (9),
- drive means for rotating the bladed rotor (8) at a speed of between 300 and 1500 revolutions per minute,
- a suction unit (13) outside the cylindrical body (2) for withdrawing the gaseous reaction products from the continuous reactor (1),
wherein said bladed rotor (8)has a plurality of blades (10) in a helical arrangement, each extending from the rotor to a position close to the hot inner wall (2a) of the continuous reactor (1).

## Patentansprüche

1. Verfahren zur kontinuierlichen Herstellung von Calciumsalzen schwacher Säuren
dadurch gekennzeichnet,
daß das Verfahren die aufeinanderfolgenden Schritte aufweist:
- kontinuierliche Zuführung von im wesentlichen wasserfreiem, pulverisiertem Calciumcarbonat und einer im wesentlichen zerstäubten schwachen Säure zu einem kontinuierlich arbeitenden Salzbildungsreaktor, der einen zylindrischen Hohlkörper mit einer inneren Wandung, aufgeheizt auf eine Temperatur zwischen 100°C und 450°C, und einen drehbar gelagerten Schaufelrotor aufweist,
- Zentrifugieren des Calciumcarbonats und der schwachen Säure, um eine stark turbulente, ringförmige Wirbelschicht auszubilden,
- Fortschreiten der ringförmigen Wirbelschicht durch den kontinuierlich arbeitenden Salzbildungsreaktor bei kontinuierlicher und gleichzeitiger Versorgung mit Hitze durch die heiße Wandung, die notwendig ist, um die Salzbildung der schwachen Säure hervorzurufen,
- kontinuierliche Ableitung der Produkte der Salzbildungsreaktion aus dem Reaktor, enthaltend Kohlendioxid, Wasserdampf und dem Calciumsalz der schwachen Säure.

2. Verfahren nach Anspruch 1, dadurch gekennzeichnet, daß die schwache Säure aus der Gruppe bestehend aus Phosphorsäure, Propionsäure und Gluconsäure ausgewählt ist.

3. Verfahren nach Anspruch 2, dadurch gekennzeichnet, daß die schwache Säure Phosphorsäure in 58-76%iger wäßriger Lösung ist und daß die innere Wandung des kontinuierlich arbeitenden Salzbildungsreaktors auf eine Temperatur zwischen 220°C und 300°C aufgeheizt ist.

4. Verfahren nach Anspruch 2, dadurch gekennzeichnet, daß die schwache Säure Propionsäure ist und die Temperatur der inneren Wandung des kontinuierlich arbeitenden Salzbildungsreaktors zwischen 130°C und 150°C liegt.

5. Verfahren nach Anspruch 2, dadurch gekennzeichnet, daß die schwache Säure Gluconsäure ist und die innere Wandung des kontinuierlich arbeitenden Salzbildungsreaktors bei einer Temperatur von 250°C gehalten wird.

6. Verfahren nach Anspruch 5, dadurch gekennzeichnet, daß die Gluconsäure in Form einer 60%igen wäßrigen Gluconsäurelacton-Lösung zugeführt wird.

7. Kontinuierlich arbeitender Reaktor für die Herstellung von Calciumsalzen von schwachen Säuren ausgehend von pulverisiertem Calciumcarbonat bestehend aus:
- einem zylindrischen Hohlkörper (2), der an den gegenüberliegenden Enden (11, 12) geschlossen ist, an denen Einlaßöffnungen (3, 4) zur Einleitung der Reagenzien und Auslaßöffnungen (5, 6) für die Reaktionsprodukte befindlich sind,
- mindestens einer Ummantelung (7), die zum Kontrollieren der Temperatur der inneren Wandung (2a) des kontinuierlichen arbeitenden Reaktors (1) bei Werten zwischen 100°C und 450°C von Heizflüssigkeit durchflossen wird,
- einem Schaufelrotor (8), der drehbar in dem Hohlkörper gelagert ist und der mit der geheizten inneren Wandung (2a) eine Reaktionskammer (9) ausbildet,
- Antriebsmitteln zum Drehen des Schaufelrotors (8) mit einer Geschwindigkeit zwischen 300 und 1500 Umdrehungen pro Minute,
- einer Saugeinheit (13) außerhalb des zylindrischen Körpers (2) zur Entfernung der gasförmigen Reaktionsprodukte aus dem kontinuierlich arbeitenden Reaktor (1),
worin der Schaufelrotor (8) eine Vielzahl von Schaufeln (10) in einer helikalen Anordnung besitzt, wobei sich jede vom Rotor in eine Position dicht zur heißen inneren Wandung (2a) des kontinuierlich arbeitenden Reaktors (1) erstreckt.

## Revendications

1. Procédé de production en continu de sels de calcium d'acides faibles caractérisé en ce qu'il comprend les étapes successives consistant à :
- alimenter en continu avec du carbonate de calcium pulvérisé substantiellement anhydre et un acide faible substantiellement atomisé un réacteur de salification en continu possédant un corps cylindrique tubulaire dont la paroi interne est chauffée à une température comprise entre 100°C et 450°C et dans lequel un rotor à pales est supporté de manière rotative ;
- centrifuger le carbonate de calcium et l'acide faible de façon à former un lit fluidisé annulaire extrêmement turbulent ;
- faire avancer le lit fluidisé annulaire à travers le réacteur de salification en continu en lui fournissant en même temps et en continu, la qualité de chaleur nécessaire pour induire la salification de l'acide faible, par l'intermédiaire de la paroi chaude ;
- décharger du réacteur en continu les produits de la réaction de salification, comprenant du dioxyde de carbone, de la vapeur d'eau et le sel de calcium de l'acide faible.

2. Procédé selon la revendication 1, caractérisé en ce que l'acide faible est choisi dans le groupe de composés comprenant les acides phosphonique, propionique et gluconique.

3. Procédé selon la revendication 2, caractérisé en ce que l'acide faible est l'acide phosphorique à 58-76 % en solution aqueuse, la paroi interne du réacteur de salification en continu étant chauffée à une température comprise entre 220°C et 300°C.

4. Procédé selon la revendication 2, caractérisé en ce que l'acide faible est l'acide propionique et en ce que la paroi interne du réacteur de salification en continu est chauffée à une température comprise entre 130°C et 150°C.

5. Procédé selon la revendication 2, caractérisé en ce que l'acide faible est l'acide gluconique et en ce que la paroi interne du réacteur de salification en continu étant maintenu à une température de 250°C.

6. Procédé selon la revendication 5, caractérisé en ce que l'acide gluconique est fourni sous forme d'une solution aqueuse à 60 % de gluconolactone.

7. Réacteur en continu pour la production de sels de calcium d'acides faibles à partir de carbonate de calcium pulvérisé, comprenant :
- un corps cylindrique tubulaire (2) fermé aux deux extrémités opposées (11, 12) et sur lequel se trouvent des ouvertures d'admission (3, 4) pour l'introduction des réactifs, et des ouvertures d'écoulement (5, 6) pour les produits de la réaction ;
- au moins une enveloppe (7) traversée par un fluide de chauffage destiné à contrôler la température de la paroi interne (2a) du réacteur en continu (1) à des valeurs comprises entre 100°C et 450°C ;
- un rotor à pales (8) supporté de manière rotative dans le corps tubulaire (2) et définissant avec la paroi interne chauffée (2a) une chambre de réaction (9);
- des moyens d'entraînement pour entraîner en rotation le rotor à pales (8) supporté de manière rotative, à une vitesse comprise entre 300 et 1500 tours par minute ;
- une unité de pompage (13) extérieure au corps cylindrique (2) pour retirer du réacteur en continu les produits gazeux de la réaction,
où ledit rotor à pales (8) possède plusieurs pales (10) dans un arrangement en hélice, chacune d'entre elles s'avançant depuis le rotor jusqu'à une position proche de la paroi interne chaude (2a) du réacteur en continu.
